(19) Europäisches Patentamt
European Patent Office
Office européen des brevets

(11) Veröffentlichungsnummer : **0 164 010**
**B1**

# (12) EUROPÄISCHE PATENTSCHRIFT

(45) Veröffentlichungstag der Patentschrift : **22.02.89**

(51) Int. Cl.⁴ : **C 07 D211/90**, C 07 D401/12, C 07 D413/12, A 61 K 31/44

(21) Anmeldenummer : **85106149.9**

(22) Anmeldetag : **20.05.85**

## (54) 3-Nitro-dihydropyridine, Verfahren zu ihrer Herstellung sowie ihre Verwendung in Arzneimitteln.

(30) Priorität : **04.06.84 DE 3420784**

(43) Veröffentlichungstag der Anmeldung : **11.12.85 Patentblatt 85/50**

(45) Bekanntmachung des Hinweises auf die Patenterteilung : **22.02.89 Patentblatt 89/08**

(84) Benannte Vertragsstaaten : **AT BE CH DE FR GB IT LI NL SE**

(56) Entgegenhaltungen :
**EP–A– 0 002 208**
**EP–A– 0 071 819**
**EP–A– 0 127 826**
**WO–A–85 /019 40**
**DE–A– 2 752 820**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Stoltefuss, Jürgen, Dipl.-Ing.**
**Parkstrasse 20**
**D-5657 Haan 2 (DE)**
Erfinder : **Heiker, Fred Robert, Dr.**
**Miles Laboratories, Inc. P.O. Box 40**
**Elkhart, IN 46515 (US)**
Erfinder : **Franckowiak, Gerhard, Dr.**
**Henselweg 10**
**D-5600 Wuppertal 1 (DE)**
Erfinder : **Schramm, Matthias, Dr.**
**Paffrather Strasse 38**
**D-5000 Köln 80 (DE)**
Erfinder : **Thomas, Günter, Dr.**
**Henselweg 5**
**D-5600 Wuppertal (DE)**
Erfinder : **Gross, Rainer, Dr.**
**Platzhoffstrasse 23**
**D-5600 Wuppertal 1 (DE)**

**EP 0 164 010 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue 3-Nitro-dihydropyridine, Verfahren zu ihrer Herstellung sowie ihre Verwendung in Arzneimitteln, insbesondere in kreislaufbeeinflussenden Arzneimitteln mit positiv inotroper Wirkung.

Es ist bereits bekannt geworden, daß 1,4-Dihydropyridine gefäßerweiternde Eigenschaften besitzen und als Coronarmittel und Antihypertensiva verwendet werden können (vgl. britisches Patent 1 173 062 ; britisches Patent 1 358 951 ; DE-OS 2 629 892 und DE-OS 2 752 820). Weiterhin ist bekannt, daß 1,4-Dihydropyridine als Calciumantagonisten eine Hemmung der Kontraktionskraft von glatten und kardialen Muskeln bewirken und zur Behandlung von Coronar- und Gefäßerkrankungen eingesetzt werden können (vgl. A. Fleckenstein, Ann. Rev. Pharmacol. Toxicol. 17, 149-166 (1977)).

Es ist weiterhin bekannt aus EP-A 0 071 819, daß bestimmte Dihydropyridine calciumagonistische bzw. kontraktionsverstärkende Wirkungen zeigen können. Bei Kenntnis dieses Standes der Technik war es nicht vorhersehbar, daß die erfindungsgemäß ausgewählten Verbindungen eine unerwartet hohe Kontraktionskraft verstärkende, am Herzmuskel positiv inotrope Wirkung besitzen. Diese positiv inotrope Wirkung ist um ein mehrfaches stärker als bei vergleichbaren bekannten Dihydropyridinen.

Die vorliegende Erfindung betrifft neue 3-Nitro-dihydropyridinderivate der allgemeinen Formel (I)

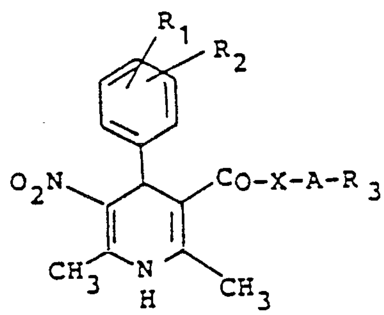

(I)

in welcher

$R_1$ für Wasserstoff steht,

$R_2$ Halogen, Trifluormethyl, Nitro, Wasserstoff, $C_1$-$C_4$-Alkyl oder eine der Gruppen

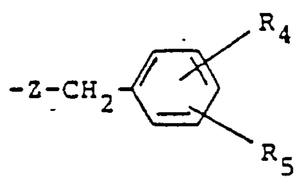

oder

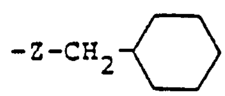

in welcher

Z Sauerstoff oder Schwefel bedeutet, und

$R_4$ und $R_5$ gleich oder verschieden sind und für Wasserstoff, $C_1$-$C_4$-Alkyl, $C_1$-$C_6$-Alkoxy, Halogen, $C_1$-$C_4$-Halogenalkyl, $C_1$-$C_4$-Halogenalkoxy oder Nitro bedeuten, oder

$R_1$ und $R_2$ gemeinsam mit 2 C-Atomen des Phenylringes den Ring

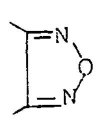

bilden,

$R_3$ für $OCOR_6$, $S—COR_6$, SH, $NH_2$, $COOR_6$, $NH—COR_6$ oder $—CO—NR_7R_8$ steht,

X Sauerstoff oder Schwefel bedeutet, und

A eine $C_2$-$C_8$-Alkylengruppe darstellt,

$R_6$ Wasserstoff oder einen aliphatischen Rest mit bis zu 6 C-Atomen oder einen Phenylrest bedeutet und

$R_7$, $R_8$ Wasserstoff oder einen aliphatischen Rest mit bis zu 6 C-Atomen oder einen Phenylrest bedeuten

sowie deren physiologisch unbedenklichen Salze.

Besonders bevorzugte Verbindungen der Formel (I) sind diejenigen, in denen

$R_1$ für Wasserstoff steht,

$R_2$ Halogen, Trifluormethyl, Nitro, Wasserstoff oder eine der Gruppen

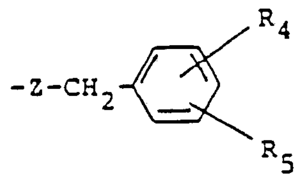

oder

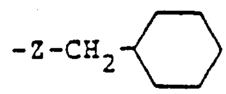

darstellt, wobei Z, $R_4$ und $R_5$ die bereits angegebenen Bedeutungen darstellen,

$R_3$ für $OCOR_6$, $S—COR_6$, SH steht,

X Sauerstoff oder Schwefel bedeutet, und

A eine $C_2$-$C_8$-Alkylengruppe darstellt.

und $R_6$ einen $C_1$-$C_3$-Alkylrest bedeutet.

Die erfindungsgemäßen Dihydropyridine der allgemeinen Formel (I) können hergestellt werden, indem man

A) Aminocrotonsäureester der allgemeinen Formel (II)

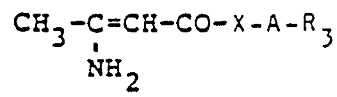

(II)

in welcher X, A und $R_3$ die oben angegebene Bedeutung haben, mit Aldehyden der allgemeinen Formel (III)

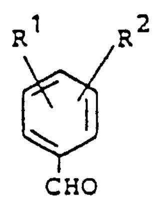

(III)

in welcher $R_1$ und $R_2$ die oben angegebene Bedeutung haben und Nitroaceton der Formel

$$CH_3—CO—CH_2—NO_2$$

umsetzt, oder

B) Benzaldehyde der Formel (III) und Acetessigsäureester der allgemeinen Formel (IV)

$$CH_3—CO—CH_2—CO—X—A—R_3 \quad (IV)$$

in welcher X, A und $R_3$ die oben angegebene Bedeutung haben bzw. deren Knoevenagel-Kondensationsprodukte (Ylidenverbindungen) der allgemeinen Formel (V)

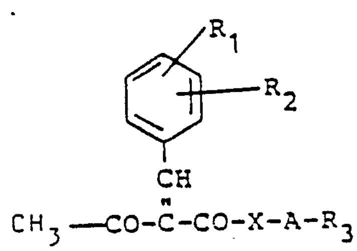

(V)

mit einer Additionsverbindung aus Nitroaceton und Ammoniak der Formel

$$CH_3—CO—CH_2—NO_2 \cdot NH_3$$

umsetzt, oder

C) indem man Aminocrotonsäureester der Formel (II) mit Benzylidennitroacetonen der allgemeinen Formel (VI)

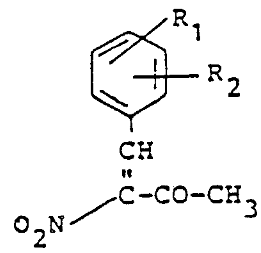

(VI)

umsetzt, oder

D) indem man Carbonsäurederivate der allgemeinen Formel

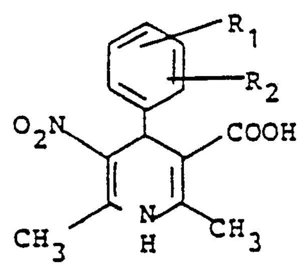

(VII)

nach bekannten Methoden gegebenenfalls über ein reaktives Säurederivat mit Verbindungen der allgemeinen Formel (VIII)

$$H—X—A—R_3 \qquad (VIII)$$

in welcher X, A und $R_3$ die oben angegebene Bedeutung haben, umsetzt, oder

E) indem man Verbindungen der Formel I, in welcher $R_1$, $R_2$, X und A die oben angegebene Bedeutung haben und $R_3$ für —$OCOR_6$, —S—$COR_6$ oder —NH—$COR_6$ steht, durch Umesterung in Verbindungen der Formel I, in welcher $R_3$ für OH, SH oder —$NH_2$ steht, überführt oder

F) indem man Verbindungen, in denen $R_1$, $R_2$, X und A die oben angegebene Bedeutung haben und $R_3$ für OH, $NH_2$ oder SH steht, durch Acylierung nach bekannten Methoden in Verbindungen mit $R_3$ gleich —O—$COR_6$, —NH—$COR_6$ oder —S—$COR_6$ überführt, oder

G) zur Herstellung von Verbindungen der allgemeinen Formel I, in welcher $R_1$, $R_2$, X und A die oben angegebene Bedeutung haben, und $R_3$ für —S—$COR_6$ oder —O—$COR_6$ steht, indem man Verbindungen der allgemeinen Formel IX

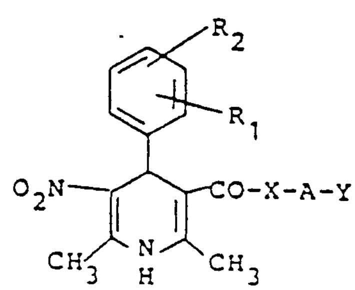

(IX)

in welcher
$R_1$, $R_2$, X und A die oben angegebene Bedeutung haben, und
Y für ein Halogenatom oder eine reaktionsfähige Gruppe wie z. B. eine Mesyl-, Tosyl-, Triflat-Gruppe steht,
mit Verbindungen der allgemeinen Formel X

$$MeR_3 \qquad (X)$$

in welcher

Me ein reaktionsfähiges Metallatom, vorzugsweise ein Alkalimetallatom bedeutet, und

$R_3$ für $—S—COR_6$ oder $—O—COR_6$ steht,

umsetzt.

Setzt man z. B. nach Verfahrensvariante A) β-Aminocrotonsäureester und Benzaldehyd mit Nitroaceton um, so läßt sich die Reaktion durch folgendes Formelschema wiedergeben :

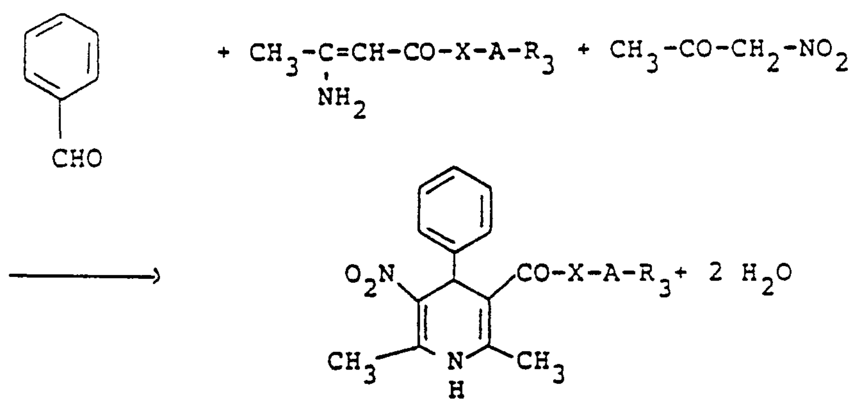

Setzt man z. B. nach Verfahrensvariante B) 2-Trifluorbenzyliden-acetessigsäureester mit Nitroaceton/Ammoniak um, so läßt sich die Reaktion durch folgendes Formelschema wiedergeben :

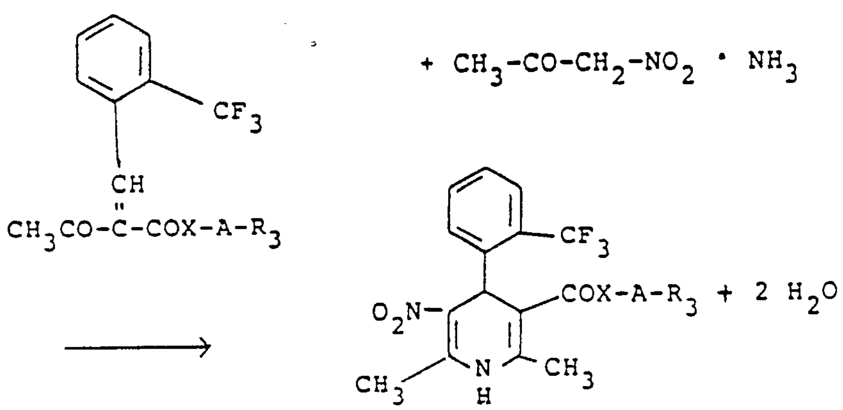

Setzt man z. B. nach Verfahrensvariante C) 2-Benzyloxybenzyliden-nitroaceton mit Aminocrotonsäureester um, so läßt sich die Reaktion durch folgendes Formelschema wiedergegeben :

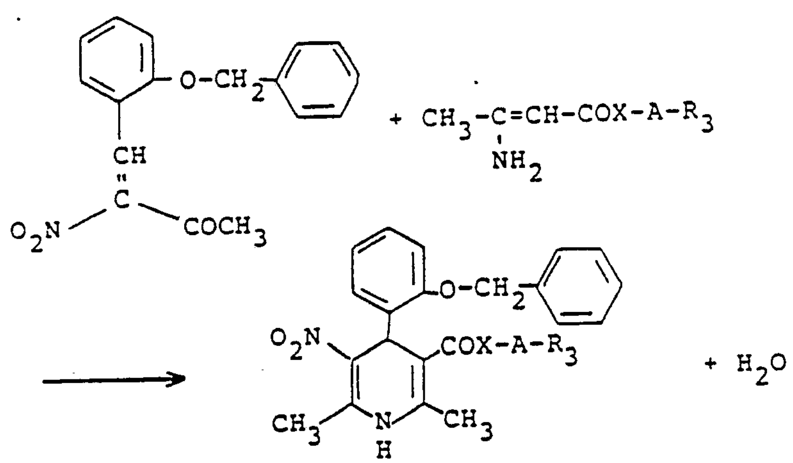

Setzt man z. B. nach Verfahrensvariante 6, 1,4-Dihydro-2,6-dimethyl-3-nitro-4(2-chlorphenyl) pyridin-5-carbonsäure-β-bromethylester mit Kaliumthioacetat um, so läßt sich die Reaktion durch folgendes Formelschema wiedergeben :

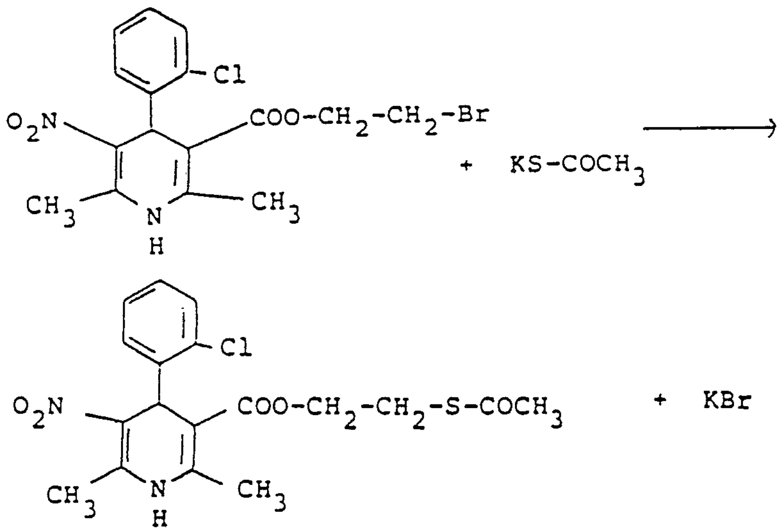

Für alle Verfahrensvarianten A, B bis G kommen als Verdünnungsmittel alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 10 und 150 °C, insbesondere zwischen 20 und 120 °C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Die vorstehenden Herstellungsverfahren sind lediglich zur Verdeutlichung angegeben, und die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Das Mengenverhältnis der Reaktanden zueinander ist beliebig, im allgemeinen werden äquimolare Mengen eingesetzt. Es hat sich jedoch als zweckmäßig erwiesen, im Verfahren A Nitroaceton und im Verfahren B das Nitroaceton/Ammoniak-Addukt im bis zu 5 molaren Überschuß einzusetzen.

Je nach der Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vergl. z. B. E. L. Eliel, Stereochemistry of Carbon Compounds, Mc Graw Hill, 1962).

Die Aminocrotonsäureester der Formel (II) sind zum Teil bekannt oder können nach bekannten Methoden aus Acetessigestern der Formel (IV) mit Ammoniak hergestellt werden, vgl. A. C. Cope, J. Am. Chem. Soc. 67, 1017 (1945).

Die Acetessigester der Formel (IV) sind zum Teil bekannt oder können nach an sich bekannten Methoden hergestellt werden (vgl. D. Borrmann, « Umsetzung von Diketen mit Alkoholen, Phenolen und Mercaptanen », in Houben-Weyl, Methoden der organischen Chemie, Vol. VII/4, 230 ff (1968) ; Y. Oikawa, K. Sugano und O. Yonemitsu, J. Org. Chem. 43, 2087 (1978)).

Die verwendeten Aldehyde (III) sind bekannt oder können nach literaturbekannten Methoden dargestellt werden (vgl. T.D. Harris und G.P. Roth, J. org. Chem. 44, 146 (1979) ; Deutsche Offenlegungsschrift 2 165 260 ; Deutsche Offenlegungsschrift 2 401 665 ; Mijano et al., Chem. Abstr. 59 (1963), 13 929 c ; E. Adler und H.-D. Becker, Chem. Scand. 15, 849 (1961) ; E.P. Papadopoulos, M. Mardin und Ch. Issidoridis, J. Org. Chem. 31, 615 (1966) J. Am. chem. Soc. 78, 2543 (1956)).

Die erfindungsgemäß verwendbaren Ylidenverbindungen der Formel (V) sind nicht bekannt, können aber nach bekannten Methoden hergestellt werden [Organic Reactions XV, 204 ff (1967)].

Die einsetzbaren Verbindungen der Formel (VI) sind bekannt und beschrieben in H. Dornoff und W. Sassenberg, Liebigs Ann. Chem. 602, 14 (1957).

Das Nitroaceton × $NH_3$ Additionsprodukt kann analog H. Böhme und K.-H. Weise Arch. Pharm., 310, 30 (1977) hergestellt werden.

Nitroaceton läßt sich nach bekannten Methoden herstellen (vgl. N. Levy u. C.W. Scarfe, J. Chem. Soc (London) (1946) 1103 ; C.D. Hurd and M.E. Nilson, J. Org. Chem. 20, 927 (1955)).

Die Verbindungen der Formeln VII bis X sind bereits bekannt oder können nach bekannten Methoden hergestellt werden, vgl. EP-OS 71 819.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt :

Wasser, nicht-toxische organische Lösungsmittel, wie Paraffine (z. B. Erdölfraktionen), pflanzliche Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin-Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebenisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Die erfindungsgemäßen Verbindungen haben eine positive inotrope Wirkung und zeigen somit ein nicht vorhersehbares und wertvolles pharmakologisches Wirkungsspektrum. Sie können als Cardiotonika zur Verbesserung der Herzkontraktilität dienen. Darüber hinaus können sie als Antihypotonika, zur Senkung von Blutzucker, zur Abschwellung von Schleimhäuten und zur Beeinflussung des Salz- und Flüssigkeitshaushaltes eingesetzt werden.

Die positiv inotrope Wirkung der erfindungsgemäßen Verbindungen der Formel (I) wird in folgender Versuchsanordnung bestimmt ; wobei besonders solche Verbindungen bevorzugt sein sollen, die bereits ab einer Konzentration von $10^{-5}$ g/ml am linken Vorhof des isolierten Meerschweinchenherzens eine positiv inotrope Wirkung zeigen :

Die linken Vorhöfe von Meerschweinchenherzen werden isoliert und in ein thermostatisiertes Organbad gehängt, welches eine isotonische Mineralsalzlösung, die dem Ionenmilieu und dem pH-Wert von Körperflüssigkeiten angepaßt ist, mit geeigneten Nährstoffen enthält. Dieses Organbad wird mit einem Gasgemisch bestehend aus Sauerstoff und Kohlendioxid begast, wobei der Kohlendioxidgehalt so bemessen ist, daß der pH-Wert der Organbades konstant bleibt. Die linken Vorhöfe werden in das Organbad eingespannt, die Spannung wird mittels eines Kraftaufnehmers registriert, wobei ein bestimmter Grundtonus eingestellt wird. Anschließend werden die linken Vorhöfe kontinuierlich in bestimmten Abständen elektrisch gereizt und die dabei erfolgenden Kontraktionen registriert. Nach Zugabe des Wirkstoffs werden die Kontraktionen weiterhin registriert. Eine Kontraktionsverstärkung um mindestens 25 % gilt als signifikante positivinotrope Wirkung.

So werden zum Beispiel die Kontraktionen des mit 1 Hz elektrisch gereizten linken Meerschweinchenvorhofs durch $10^{-5}$ g/ml der Verbindung aus Beispiel 28 um 65 % und durch die Verbindung aus Beispiel 34 um 94 % verstärkt.

Ausführungsbeispiele

Beispiel 1 (Verfahrensvariante A)

1,4-Dihydro-2,6-dimethyl-3-nitro-4-(2-chlorphenyl)-pyridin-5-carbonsäure-β-acetoxyethylester

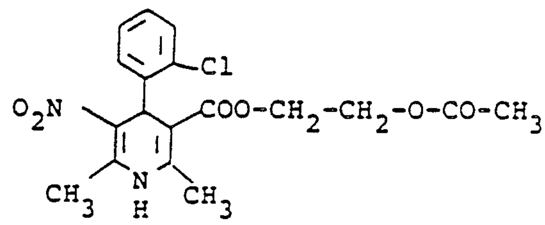

28,1 g (200 mmol) 2-Chlorbenzaldehyd werden mit 37,4 g (200 mmol) β-Aminocrotonsäure-2-acetoxyethylester und 32,6 g (320 mmol) Nitroaceton in 400 ml Ethanol 4 Stunden zum Rückfluß gekocht. Es wird abgekühlt und eingeengt. Der Eindampfrückstand wird in Essigester aufgenommen, es wird mit Wasser, Natriumhydrogencarbonatlösung und wieder Wasser gewaschen, getrocknet und eingeengt. Der halbfeste Eindampfrückstand wird mit Ethanol verrührt, abgesaugt und mit Ethanol gewaschen. Man erhält 23,2 g (29,4 % der Theorie) eines gelbgefärbten Produktes vom Schmp. : 158-160 °C.

Analog werden hergestellt :

Beispiel 2

1,4-Dihydro-2,6-dimethyl-3-nitro-4-(2-benzyloxy-phenyl)-pyridin-5-carbonsäure-β-acetoxyethylester vom Schmp : 199 °C.

Beispiel 3

1,4-Dihydro-2,6-dimethyl-3-nitro-4-phenyl-pyridin-5-carbonsäure-β-acetoxyethylester vom Schmp. : 163-65 °C;

Beispiel 4

1,4-Dihydro-2,6-dimethyl-3-nitro-4-(2-benzyloxy-phenyl)-pyridin-5-carbonsäure-2-acetylthioethylester vom Schmp. : 152-54 °C.

Beispiel 5

1,5-Dihydro-2,6-dimethyl-3-nitro-4-(2-benzyloxy-phenyl)-pyridin-5-carbonsäure-3-acetylthio-propylester vom Schmp. : 185-187 °C.

Beispiel 6

1,4-Dihydro-2,6-dimethyl-3-nitro-4-(2-benzyloxy-phenyl)-pyridin-5-carbonsäure-β-phthalimido-ethylester vom Schmp. : 225 °C.

Beispiel 7

1,4-Dihydro-2,6-dimethyl-3-nitro-4-(2-benzyloxy-phenyl)-pyridin-5-carbonsäure-6-phthalimido-hexylester vom Schmp. : 224 °C.

Beispiel 8

1,4-Dihydro-2,6-dimethyl-3-nitro-4-(2-benzyloxy-phenyl)-pyridin-5-carbonsäure-5-phthalimido-pentylester vom Schmp. : 164 °C.

Beispiel 9

1,4-Dihydro-2,6-dimethyl-3-nitro-4-[2-(4-methyl-benzyloxy)-phenyl]-pyridin-5-carbonsäure-β-acetoxy-ethylthioester vom Schmp. : 135-37 °C.

Beispiel 10

1,4-Dihydro-2,6-dimethyl-3-nitro-4-(2-fluorphenyl)-pyridin-5-carbonsäure-β-acetoxy-ethylthioester, isoliert als Schaum, Rf-Wert 0,45.

Falls nicht ausdrücklich anders angegeben, werden dieser und alle folgenden Rf-Werte ermittelt an : DC-Alurolle, Merck, Kieselgel 60 $F_{254}$ ; Fließmittel : Toluol : Essigester (Vol. 1 : 1).

Beispiel 11

1,4-Dihydro-2,6-dimethyl-3-nitro-4-(2-nitrophenyl)-pyridin-5-carbonsäure-β-acetylthio-ethylester, isoliert als Schaum, Rf-Wert : 0,30.

Beispiel 12

1,4-Dihydro-2,6-dimethyl-3-nitro-4-(2-fluorphenyl)-pyridin-5-carbonsäure-β-acetylthio-ethylester vom Schmp. : 140-143 °C.

Beispiel 13

1,4-Dihydro-2,6-dimethyl-3-nitro-4-(2-chlorphenyl)-pyridin-5-carbonsäure-β-acetoxy-ethylthioester, isoliert als Schaum.

Rf-Wert 0.364.

Beispiel 14

1,4-Dihydro-2,6-dimethyl-3-nitro-4-(4-chlorphenyl)-pyridin-5-carbonsäure-β-acetoxy-ethylthioester, isoliert als Schaum, Rf-Wert : 0,386.

Beispiel 15

1,4-Dihydro-2,6-dimethyl-3-nitro-4-(3-chlorphenyl)-pyridin-5-carbonsäure-β-acetoxyethylester vom Schmp. : 122-24 °C.

Beispiel 16

1,4-Dihydro-2,6-dimethyl-3-nitro-4-(2-fluorphenyl)-pyridin-5-carbonsäure-β-acetoxyethylester vom Schmp. : 182-184 °C.

Beispiel 17

Verfahrensvariante D

1,4-Dihydro-2,6-dimethyl-3-nitro-4-(2-chlorphenyl)-pyridin-5-carbonsäure-β-hydroxy-ethylthioester.

3 g 1,4-Dihydro-2,6-dimethyl-3-nitro-4-(2-chlorphenyl)-pyridin-5-carbonsäureimidazolid werden in 12,5 ml absolutem THF mit 5 ml Mercaptoethanol 7 Stunden gekocht. Es wird eingeengt, an der Ölpumpe bei 70 °C eingeengt, in Chloroform gelöst, 2 x mit Wasser gewaschen, getrocknet und eingeengt. Der erhaltene Eindampfrückstand wird über eine Kieselgelsäule von 160 ml Volumen mit Toluol, später Toluol/Essigester 6 : 1 als mobiler Phase gereinigt. Als Hauptfraktion werden 1.94 g eines gelben Schaumes mit einem Rf-Wert von 0,25 erhalten.

Als Nebenprodukt wird

Beispiel 18

1,4-Dihydro-2,6-dimethyl-3-nitro-4-(2-chlorphenyl)-pyridin-5-carbonsäure-β-mercaptoethylester, Rf-Wert 0,55, isoliert.

Analog Beispiel 17 werden erhalten

Beispiel 19

1,4-Dihydro-2,6-dimethyl-3-nitro-4-(3-chlorphenyl)-pyridin-5-carbonsäure-β-mercaptoethylester, Rf-Wert 0,54.

Beispiel 20

1,4-Dihydro-2,6-dimethyl-3-nitro-4-(3-chlorphenyl)-pyridin-5-carbonsäure-β-hydroxy-ethylthioester, Rf-Wert 0,24.

Beispiel 21

1,4-Dihydro-2,6-dimethyl-3-nitro-4-(2-fluorphenyl)-pyridin-5-carbonsäure-β-hydroxy-ethylthioester, Rf-Wert 0,25.

Beispiel 22

1,4-Dihydro-2,6-dimethyl-3-nitro-4-[2-(4-chlor-benzyl-oxy)-phenyl]-pyridin-5-carbonsäure-β-hydroxy-ethylthioester vom Schmp. : 186 °C.

Beispiel 23

1,4-Dihydro-2,6-dimethyl-3-nitro-4-phenyl-pyridin-5-carbonsäure-β-hydroxy-ethylthioester, isoliert als Öl, Rf-Wert 0,21.

Beispiel 24

1,4-Dihydro-2,6-dimethyl-3-nitro-4-[2-(4-methyl-benzyloxy)-phenyl]-pyridin-5-carbonsäure-β-hydroxy-ethylthioester vom Schmp. : 186-90 °C.

Herstellungsbeispiel Nr. 25 (Verfahrensvariante F)

1,4-Dihydro-2,6-dimethyl-3-nitro-4-(2-chlorphenyl)-pyridin-5-carbonsäure-β-acetoxyethylester.

1 g 1,4-Dihydro-2,6-dimethyl-3-nitro-4-(2-chlorphenyl)-pyridin-5-carbonsäure-β-hydroxyethylester werden in einer Mischung von 15 ml Pyridin und 15 ml Acetanhydrid über Nacht gerührt. Es wird in Wasser gegossen, mit Essigester extrahiert, mit 1 N HCl, Wasser, Natriumhydrogencarbonatlösung und wieder Wasser gewaschen, getrocknet und eingeengt. Der Eindampfrückstand wird mit Ethanol kristallisiert, abgesaugt und mit Ethanol gewaschen. Man erhält 800 mg einer gelbgefärbten Verbindung vom Schmp. 166 °C, die identisch ist mit der Verbindung aus dem Herstellungsbeispiel 1, Verfahrensvariante A.

Analog werden hergestellt :

Beispiel 26

1,4-Dihydro-2,6-dimethyl-3-nitro-4-(2-benzyloxyphenyl)-pyridin-5-carbonsäure-(5-acetylamino-pentylester) vom Schmp. : 191 °C.

Beispiel 27

1,4-Dihydro-2,6-dimethyl-3-nitro-4-(2-benzyloxyphenyl)-pyridin-5-carbonsäure-β-acetylaminoethylester vom Schmp. : 252 °C.

Herstellungsbeispiel 28 (Verfahrensvariante E)

1,4-Dihydro-2,6-dimethyl-3-nitro-4-(2-benzyloxyphenyl)-pyridin-5-carbonsäure-β-aminoethylester.

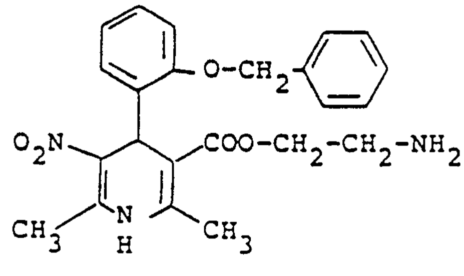

14,5 g (26,22 mmol), 1,4-Dihydro-2,6-dimethyl-3-nitro-4-(2-benzoyloxyphenyl)-pyridin-5-carbonsäure-β-phthalimido-ethylester werden in 260 ml Ethanol suspendiert und unter Kochen tropfenweise mit 7 ml

(140 mmol) Hydrazinhydrat in 20 ml Ethanol versetzt. Es wird 2 Std. gekocht, abgekühlt und abgesaugt. Das erhaltene Festprodukt wird mit 200 mmol 0,5 N Natronlauge 30 Minuten verrührt, abgesaugt, mit Wasser und Ethanol gewaschen und getrocknet. Man erhält 9,5 g rotorange gefärbte Kristalle vom Schmp. : 216 °C.

Analog werden hergestellt :

Beispiel 29

1,4-Dihydro-2,6-dimethyl-3-nitro-4-(2-benzyloxy-phenyl)-pyridin-5-carbonsäure-(6-aminohexyl)ester vom Schmp. : 146 °C.

Beispiel 30

1,4-Dihydro-2,6-dimethyl-3-nitro-4-(2-benzyloxy-phenyl)-pyridin-5-carbonsäure-(5-amino-pentyl)-ester vom Schmp. : 148 °C.

Beispiel 31

Verfahrensvariante C

1,4-Dihydro-2,6-dimethyl-3-nitro-4-(2-benzyloxy-phenyl)-pyridin-5-carbonsäure-ethoxycarbonylethyl-mercapto-ethylester.

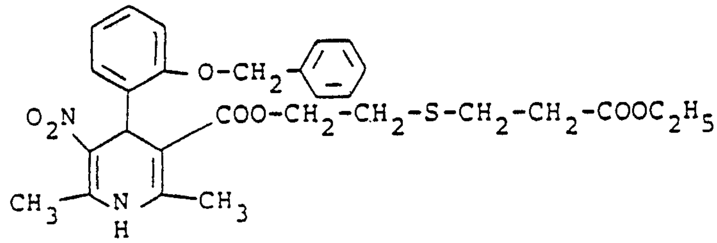

12 g (40 mmol) 2-Benzyloxybenzyliden-nitroaceton werden mit 10,5 g β-Aminocrotonsäure-ethoxycarbonyl-ethylmercaptoethylester in 80 ml Ethanol 4 Std. gekocht. Es wird eingeengt und über eine Kieselgelsäule mit Toluol/Essigester im Volumenverhältnis 10 : 1 gereinigt. Die Fraktionen mit dem sauberen Produkt werden vereint und eingeengt. Nach Umkristallisation aus Ethanol erhält man 10,2 g orangefarbene Kristalle vom Schmelzpunkt 133 °C.

Beispiel 32

1,4-Dihydro-2,6-dimethyl-3-nitro-4-(2-benzyloxy-phenyl)-pyridin-5-carbonsäure-ethoxycarbonylpropylmercaptoethylester vom Schmp. : 121 °C.

Durch Hydrolyse der Verbindung aus Beispiel 31 erhält man :

Beispiel 33

1,4-Dihydro-2,6-dimethyl-3-nitro-4-(2-benzyloxy-phenyl)-pyridin-5-carbonsäure-hydroxycarbonylethylmercaptoethylester vom Schmp. : 178 °C.

Beispiel 34 (aus Beispiel 32)

1,4-Dihydro-2,6-dimethyl-3-nitro-4-(2-benzyloxy-phenyl)-pyridin-5-carbonsäure-hydroxycarbonyl-propylmercaptoethylester vom Schmp. : 160 °C.

Beispiel 35

Verfahrensvariante G

1,4-Dihydro-2,6-dimethyl-3-nitro-4-(2-benzyloxyphenyl)-pyridin-5-carbonsäure-2-(2-acetylthio-ethoxy)-ethyl ester

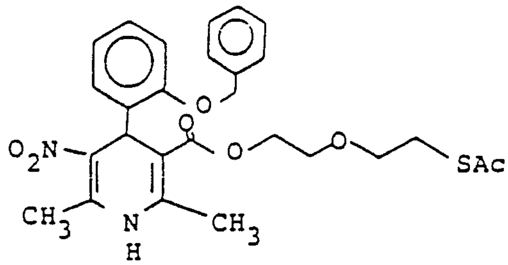

1,3 g 1,4-Dihydro-2,6-dimethyl-3-nitro-4-(2-benzyloxy-phenyl)-pyridin-5-carbonsäure-2(2-chloroethoxy)-ethyl ester werden in 15 ml DMF gelöst und nach Zugabe von 500 mg Kaliumthioacetat über Nacht bei 100 °C gerührt. Nach beendeter Reaktion wird der Ansatz am Rotationsverdampfer eingeengt, der Rückstand in Chloroform aufgenommen, die organische Phase mit Wasser gewaschen, getrocknet und eingeengt. Der kristalline Rückstand wird aus Essigester/Petrolether umkristallisiert.

Ausbeute : 1,1 g (78 %) Fp. 167 °C

Rf (Tol/Aceton 4 : 1) : 0,46

Rf (Tol/Essigester (EE) 6 : 1) : 0,39

Analog werden hergestellt :

Beispiel 36

1,4-Dihydro-2,6-dimethyl-3-nitro-4-(2-benzyloxyphenyl)-pyridin-5-carbonsäure-(4-acetylthio) butylester

Rf (Tol/Ac 4 : 1) : 0,48 Rf (Tol/EE 6 : 1) : 0,39

Beispiel 37

1,4-Dihydro-2,6-dimethyl-3-nitro-4-(2-benzyloxyphenyl)-pyridin-5-carbonsäure-(6-acetylthio) hexylester

Rf (Tol/Ac 4 : 1) : 0,54 Rf (Tol/EE 8 : 1) : 0,41

Beispiel 38

1,4-Dihydro-2,6-dimethyl-3-nitro-4-(2-benzyloxyphenyl)-pyridin-5-carbonsäure-(8-acetylthio-3,6-dioxo)-octylester

Rf (Tol/Ac 4 : 1) : 0,42 Rf(Tol/EE 6 : 1) : 0,39

Beispiel 39 (Darstellung nach Verfahrensvariante G)

1,4-Dihydro-2,6-dimethyl-3-nitro-4-(2-benzyloxyphenyl)-pyridin-5-carbonsäure-(11-acetylthio)undecylester

Rf (Tol/Ac 4 : 1) : 0,59 Rf (Tol/EE 6 : 1) : 0,43

Beispiel 40

1,4-Dihydro-2,6-dimethyl-3-nitro-4-(2-benzyloxy-phenyl)-pyridin-5-carbonsäure-(2-dimethylaminoethylmercapto)-ethylester vom Schmp. : 125 °C

(Rf-Wert DC-Alurolle, Merck, Kieselgel 60 F 254

Fließmittel : Chloroform/Methanol im Volumenverhältnis 3 : 1)

Rf-Wert : 0,66

Beispiel 41

1,4-Dihydro-2,6-dimethyl-3-nitro-4-(2-benzyloxy-phenyl)-pyridin-5-carbonsäure-(3-dimethylaminoethylmercapto)-propylester vom Schmp. : 127 °C

(Rf-Wert DC-Alurolle, Merck, Kieselgel 60 F 254

Fließmittel : Chloroform/Methanol im Volumenverhältnis 3 : 1)

Rf-Wert : 0,43.

Beispiel 42

1,4-Dihydro-2,6-dimethyl-3-nitro-4-(3-chlorphenyl)-pyridin-5-carbonsäure-3-acetylmercaptopropylester vom Schmp. : 125°-127 °C.

Beispiel 43

1,4-Dihydro-2,6-dimethyl-3-nitro-4-(2-fluorphenyl)-pyridin-5-carbonsäure-3-acetylmercaptopropylester vom Schmp. : 125°-130 °C.

Beispiel 44

1,4-Dihydro-2,6-dimethyl-3-nitro-4-(3-chlorphenyl)-pyridin-5-carbonsäure-β-acetylmercaptoethylester vom Schmp. : 102°-104 °C.

Beispiel 45

1,4-Dihydro-2,6-dimethyl-3-nitro-4-(3-methylphenyl)-pyridin-5-carbonsäure-β-acetylmercaptoethylester vom Schmp. : 150 °C.

Beispiel 46

1,4-Dihydro-2,6-dimethyl-3-nitro-4-(3-methylphenyl)-pyridin-5-carbonsäure-3-acetylmercaptopropylester vom Schmp. : 184 °C.

Beispiel 47

1,4-Dihydro-2,6-dimethyl-3-nitro-4-(2-chlorphenyl)-pyridin-5-carbonsäure-3-acetylmercaptopropylester vom Schmp. : 197 °C.

Beispiel 48

1,4-Dihydro-2,6-dimethyl-3-nitro-4-(2-methylphenyl)-pyridin-5-carbonsäure-β-acetylmercaptoethylester vom Schmp. : 180 °C.

Beispiel 49

1,4-Dihydro-2,6-dimethyl-3-nitro-4-(2,3-dichlorphenyl)-pyridin-5-carbonsäure-β-acetylmercaptoethylester.

Beispiel 50

1,4-Dihydro-2,6-dimethyl-3-nitro-4-(2,3-dichlorphenyl)-pyridin-5-carbonsäure-3-acetylmercaptopropylester.

Beispiel 51

1,4-Dihydro-2,6-dimethyl-3-nitro-4-(3-chlorphenyl)-pyridin-5-carbonsäure-3-mercaptopropylester.
Rf-Wert = 0,5
DC-Alurolle Merck, Kieselgel 60 F 254 :
Laufmittel Chloroform/Essigester im Volumenverhältnis 6 : 1

Beispiel 52

1,4-Dihydro-2,6-dimethyl-3-nitro-4-(2-fluorphenyl)-pyridin-5-carbonsäure-3-mercaptopropylester.
Rf-Wert : 0,5

Beispiel 53

1,4-Dihydro-2,6-dimethyl-3-nitro-4-(3-methylphenyl)-pyridin-5-carbonsäure-β-mercaptoethylester.
Rf-Wert : 0,49

Beispiel 54

1,4-Dihydro-2,6-dimethyl-3-nitro-4-(3-methylphenyl)-pyridin-5-carbonsäure-3-mercaptopropylester.
Rf-Wert : 0,49

Beispiel 55

1,4-Dihydro-2,6-dimethyl-3-nitro-4-(2-chlorphenyl)-pyridin-5-carbonsäure-3-mercaptopropylester.
Rf-Wert : 0,46

Beispiel 56

1,4-Dihydro-2,6-dimethyl-3-nitro-4-(2-methylphenyl)-pyridin-5-carbonsäure-2-mercaptoethylester.
Rf-Wert : 0,52

Beispiel 57

1,4-Dihydro-2,6-dimethyl-3-nitro-4-(2,3-dichlorphenyl)-pyridin-5-carbonsäure-2-mercaptoethylester.

Beispiel 58

1,4-Dihydro-2,6-dimethyl-3-nitro-4-(2,3-dichlorphenyl)-pyridin-5-carbonsäure-3-mercaptopropylester.

**Patentansprüche**

1. 3-Nitro-dihydropyridinderivate der allgemeinen Formel (I)

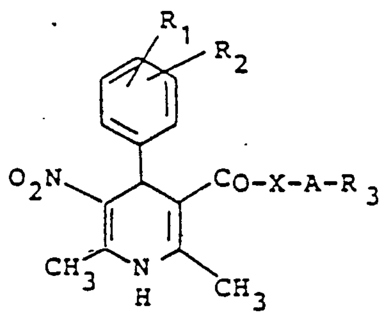

(I)

in welcher
$R_1$ für Wasserstoff steht,
$R_2$ Halogen, Trifluormethyl, Nitro, Wasserstoff, $C_1$-$C_4$-Alkyl oder eine der Gruppen

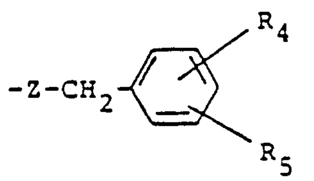

oder

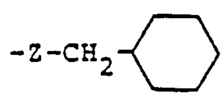

in welcher
Z Sauerstoff oder Schwefel bedeutet, und
$R_4$ und $R_5$ gleich oder verschieden sind und für Wasserstoff, $C_1$-$C_4$-Alkyl, $C_1$-$C_6$-Alkoxy, Halogen, $C_1$-$C_4$-Halogenalkyl, $C_1$-$C_4$-Halogenalkoxy oder Nitro bedeuten, oder
$R_1$ und $R_2$ gemeinsam mit 2 C-Atomen des Phenylringes den Ring

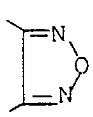

bilden,
$R_3$ für $OCOR_6$, $S—COR_6$, SH, $NH_2$, $COOR_6$, $NH—COR_6$ oder $—CO—NR_7R_8$ steht,
X Sauerstoff oder Schwefel bedeutet, und
A eine $C_2$-$C_8$-Alkylengruppe darstellt,
$R_6$ Wasserstoff oder einen aliphatischen Rest mit bis zu 6 C-Atomen oder einen Phenylrest bedeutet und
$R_7$, $R_8$ Wasserstoff oder einen aliphatischen Rest mit bis zu 6 C-Atomen oder einen Phenylrest bedeuten,
sowie deren physiologisch unbedenklichen Salze.

2. Verbindungen der allgemeinen Formel (I) gemäß Anspruch 1, in welcher
$R_1$ für Wasserstoff steht,
$R_2$ Halogen, Trifluormethyl, Nitro, Wasserstoff oder eine der Gruppen

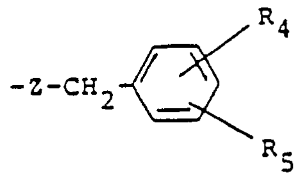

oder

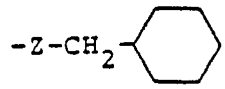

darstellt, wobei

Z, $R_4$ und $R_5$ die im Anspruch 1 angegebene Bedeutung haben,

$R_3$ für $OCOR_6$, $S—COR_6$, SH steht,

X Sauerstoff oder Schwefel bedeutet, und

A eine $C_2$-$C_8$-Alkylengruppe darstellt

und $R_6$ einen $C_1$-$C_3$-Alkylrest bedeutet.

3. Verbindungen der allgemeinen Formel I gemäß Anspruch 1 zur Verwendung bei der Bekämpfung von Kreislauferkrankungen.

4. Verfahren zur Herstellung von Verbindungen der allgemeinen Formel (I)

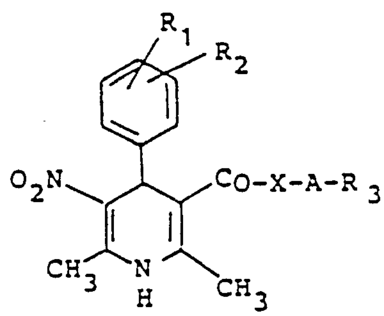

(I)

in welcher

$R_1$ für Wasserstoff steht,

$R_2$ Halogen, Trifluormethyl, Nitro, Wasserstoff, $C_1$-$C_4$-Alkyl oder eine der Gruppen

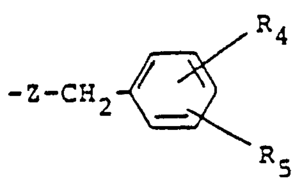

oder

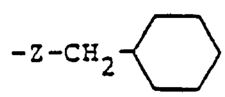

in welcher

Z Sauerstoff oder Schwefel bedeutet, und

$R_4$ und $R_5$ gleich oder verschieden sind und für Wasserstoff, $C_1$-$C_4$-Alkyl, $C_1$-$C_6$-Alkoxy, Halogen, $C_1$-$C_4$-Halogenalkyl, $C_1$-$C_4$-Halogenalkoxy oder Nitro bedeuten, oder

$R_1$ und $R_2$ gemeinsam mit 2 C-Atomen des Phenylringes den Ring

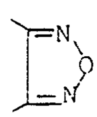

bilden,

$R_3$ für $OCOR_6$, $S—COR_6$, SH, $NH_2$, $COOR_6$, $NH—COR_6$ oder $—CO—NR_7R_8$ steht,

X Sauerstoff oder Schwefel bedeutet, und

A eine $C_2$-$C_8$-Alkylengruppe darstellt,

$R_6$ Wasserstoff oder einen aliphatischen Rest mit bis zu 6 C-Atomen oder einen Phenylrest bedeutet und

$R_7$, $R_8$ Wasserstoff oder einen aliphatischen Rest mit bis zu 6 C-Atomen oder einen Phenylrest bedeuten,

sowie deren physiologisch unbedenklichen Salze, dadurch gekennzeichnet, daß man

A) Aminocrotonsäureester der allgemeinen Formel

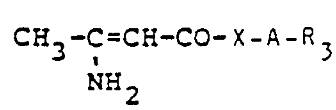

(II)

in welcher X, A und $R_3$ die oben angegebene Bedeutung haben, mit Aldehyden der allgemeinen Formel (III)

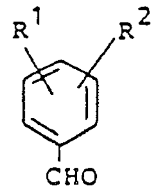

(III)

in welcher $R_1$ und $R_2$ die oben angegebene Bedeutung haben und Nitroaceton der Formel

$$CH_3—CO—CH_2—NO_2$$

umsetzt, oder

B) Benzaldehyde der Formel (III) und Acetessigsäureester der allgemeinen Formel (IV)

$$CH_3—CO—CH_2—CO—X—A—R_3 \qquad (IV)$$

in welcher X, A und $R_3$ die oben angegebene Bedeutung haben bzw. deren Knoevenagel-Kondensationsprodukte (Ylidenverbindungen) der allgemeinen Formel (V)

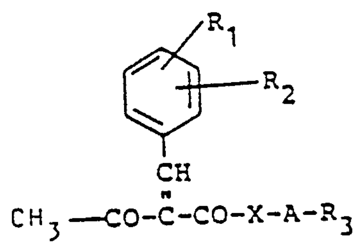

(V)

mit einer Additionsverbindung aus Nitroaceton und Ammoniak der Formel

$$CH_3—CO—CH_2—NO_2 \cdot NH_3$$

umsetzt, oder

C) indem man Aminocrotonsäureester der Formel (II) mit Benzylidennitroacetonen der allgemeinen Formel (VI)

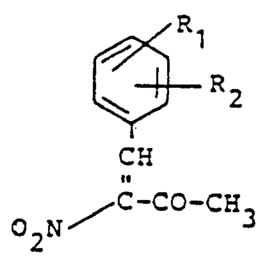

(VI)

umsetzt, oder

D) indem man Carbonsäurederivate der allgemeinen Formel

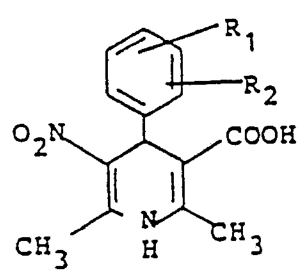

(VII)

nach bekannten Methoden gegebenenfalls über ein reaktives Säurederivat mit Verbindungen der allgemeinen Formel (VIII)

$$H—X—A—R_3 \quad (VIII)$$

in welcher X, A und $R_3$ die oben angegebene Bedeutung haben, umsetzt, oder

E) indem man Verbindungen der Formel I, in welcher $R_1$, $R_2$, X und A die oben angegebene Bedeutung haben und $R_3$ für —$OCOR_6$, —S—$COR_6$ oder —NH—$COR_6$ steht, durch Umesterung in Verbindungen der Formel I, in welcher $R_3$ für OH, SH oder —$NH_2$ steht, überführt oder

F) indem man Verbindungen, in denen $R_1$, $R_2$, X und A die oben angegebene Bedeutung haben und $R_3$ für OH, $NH_2$ oder SH steht, durch Acylierung nach bekannten Methoden in Verbindungen mit $R_3$ gleich —O—$COR_6$, —NH—$COR_6$ oder —S—$COR_6$ überführt, oder

G) zur Herstellung von Verbindungen der allgemeinen Formel I, in welcher $R_1$, $R_2$, X und A die oben angegebene Bedeutung haben, und $R_3$ für —S—$COR_6$ oder —O—$COR_6$ steht, Verbindungen der allgemeinen Formel IX

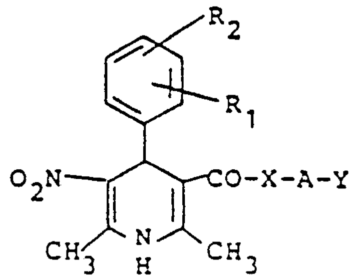

(IX)

in welcher

$R_1$, $R_2$, X und A die oben angegebene Bedeutung haben, und

Y für ein Halogenatom oder eine reaktionsfähige Gruppe wie z. B. eine Mesyl-, Tosyl-, Triflat-Gruppe steht,

mit Verbindungen der allgemeinen Formel X

$$MeR_3 \quad (X)$$

in welcher

Me ein reaktionsfähiges Metallatom, vorzugsweise ein Alkalimetallatom bedeutet, und

$R_3$ für —S—$COR_6$ oder —O—$COR_6$ steht,

umsetzt.

5. Verfahren gemäß Anspruch 3, dadurch gekennzeichnet, daß man die Umsetzung bei Temperaturen zwischen 10 und 150 °C durchführt.

6. Arzneimittel enthaltend mindestens eine Verbindung der allgemeinen Formel (I) gemäß Anspruch 1.

7. Kreislaufbeeinflussende Arzneimittel mit positiv inotroper Wirkung, enthaltend mindestens eine Verbindung der allgemeinen Formel (I) gemäß Anspruch 1.

8. Verfahren zur Herstellung von Arzneimitteln gemäß den Ansprüchen 5 und 6, dadurch gekennzeichnet, daß man Verbindungen der allgemeinen Formel (I) gemäß Anspruch 1 gegebenenfalls unter Verwendung üblicher Hilfs- und Trägerstoffe in eine geeignete Applikationsform überführt.

9. Verwendung von Verbindungen der allgemeinen Formel (I) gemäß Anspruch 1 zur Herstellung von Arzneimitteln zur Bekämpfung von Kreislauferkrankungen.

**Claims**

1. 3-Nitro-dihydropyridine derivatives of the general formula (I)

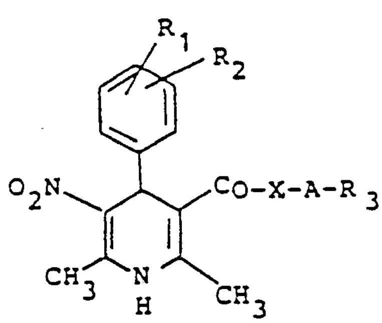

(I)

in which

$R_1$ represents hydrogen,

$R_2$ represents halogen, trifluoromethyl, nitro, hydrogen, $C_1$-$C_4$-alkyl or one of the groups

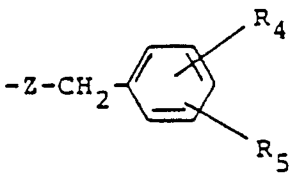

or

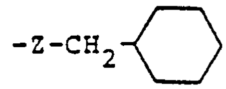

in which

Z denotes oxygen or sulphur and

$R_4$ and $R_5$ are identical or different and denote hydrogen, $C_1$-$C_4$-alkyl, $C_1$-$C_6$-alkoxy, halogen, $C_1$-$C_4$-halogenoalkyl, $C_1$-$C_4$-halogenoalkoxy or nitro, or

$R_1$ and $R_2$, together with 2 C atoms of the phenyl ring, form the ring

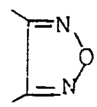

$R_3$ represents $OCOR_6$, $S—COR_6$, SH, $NH_2$, $COOR_6$, $NH—COR_6$ or $—CO—NR_7R_8$,

X denotes oxygen or sulphur and

A represents a $C_2$-$C_8$-alkylene group,

$R_6$ denotes hydrogen or an aliphatic radical with up to 6 C atoms or a phenyl radical and

$R_7$ and $R_8$ denote hydrogen or an aliphatic radical with up to 6 C atoms or a phenyl radical, and physiologically acceptable salts thereof.

2. Compounds of the general formula (I) according to Claim 1, in which

$R_1$ represents hydrogen,

$R_2$ represents halogen, trifluoromethyl, nitro, hydrogen or one of the groups

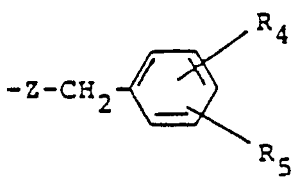

or

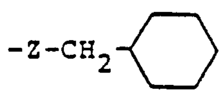

wherein

Z, $R_4$ and $R_5$ have the meaning given in Claim 1,

$R_3$ represents $OCOR_6$, $S—COR_6$ or SH,

X denotes oxygen or sulphur and

A represents a $C_2$-$C_8$-alkylene group, and

$R_6$ denotes a $C_1$-$C_3$-alkyl radical.

3. Compounds of the general formula I according to Claim 1 for use in combating circulating diseases.

4. Process for the preparation of compounds of the general formula (I)

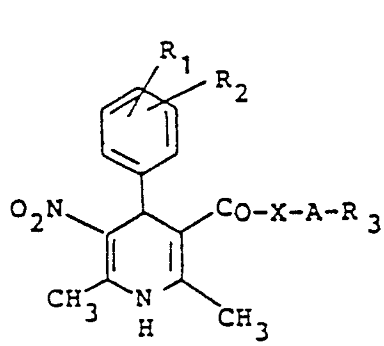

(I)

in which

$R_1$ represents hydrogen,

$R_2$ represents halogen, trifluoromethyl, nitro, hydrogen, $C_1$-$C_4$-alkyl or one of the groups

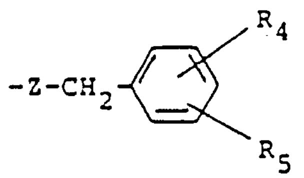

or

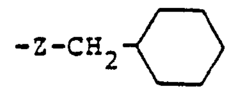

in which

Z denotes oxygen or sulphur and

$R_4$ and $R_5$ are identical or different and denote hydrogen, $C_1$-$C_4$-alkyl, $C_1$-$C_6$-alkoxy, halogen, $C_1$-$C_4$-halogenoalkyl, $C_1$-$C_4$-halogenoalkoxy or nitro, or

$R_1$ and $R_2$, together with 2 C atoms of the phenyl ring, form the ring

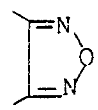

$R_3$ represents $OCOR_6$, $S—COR_6$, SH, $NH_2$, $COOR_6$, $NH—COR_6$ or $—CO—NR_7R_8$,

X denotes oxygen or sulphur and

A represents a $C_2$-$C_8$-alkylene group,

$R_6$ denotes hydrogen or an aliphatic radical with up to 6 C atoms or a phenyl radical and

$R_7$ and $R_8$ denote hydrogen or an aliphatic radical with up to 6 C atoms or a phenyl radical,

and physiologically acceptable salts thereof, characterised in that

A) aminocrotonic acid esters of the general formula

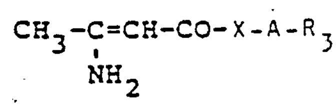

(II)

in which X, A and $R_3$ have the abovementioned meaning, are reacted with aldehydes of the general formula (III)

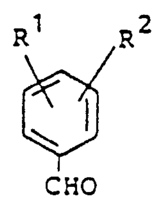

(III)

in which $R_1$ and $R_2$ have the abovementioned meaning, and nitroacetone of the formula

$$CH_3—CO—CH_2—NO_2$$

or

B) benzaldehydes of the formula (III) and acetoacetic acid esters of the general formula (IV)

$$CH_3—CO—CH_2—CO—X—A—R_3 \quad \text{(IV)}$$

in which X, A and $R_3$ have the abovementioned meaning, or Knoevenagel condensation products thereof (ylidene compounds) of the general formula (V)

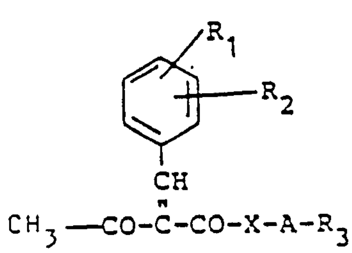

(V)

are reacted with an addition compound of nitroacetone and ammonia, of the formula

$$CH_3—CO—CH_2—NO_2 \cdot NH_3$$

or

C) by a process in which aminocrotonic acid esters of the formula (II) are reacted with benzylidenenitroacetones of the general fomula (VI)

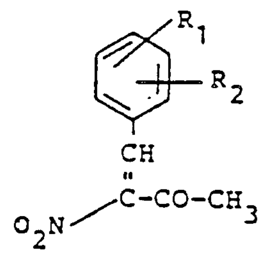

(VI)

or

D) by a process in which carboxylic acid derivatives of the general formula

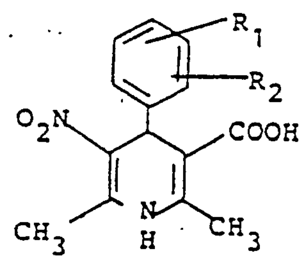

(VII)

are reacted with compounds of the general formula (VIII)

$$H—X—A—R_3 \quad (VIII)$$

in which X, A and $R_3$ have the abovementioned meaning, by known methods, if appropriate via a reactive acid derivative, or

E) by a process in which compounds of the formula I in which $R_1$, $R_2$, X and A have the abovementioned meaning and $R_3$ represents $—OCOR_6$, $—S—COR_6$ or $—NH—COR_6$, are converted into compounds of the formula I in which $R_3$ represents OH, SH or $—NH_2$ by esterification, or

F) by a process in which compounds in which $R_1$, $R_2$, X and A have the abovementioned meaning and $R_3$ represents OH, $NH_2$ or SH, are converted into compounds where $R_3$ is $—O—COR_6$, $—NH—COR_6$ or $—S—COR_6$ by acylation by known methods, or

G) to prepare compounds of the general formula I in which $R_1$, $R_2$, X and A have the abovementioned meaning and $R_3$ represents $—S—COR_6$ or $—O—COR_6$, compounds of the general formula IX

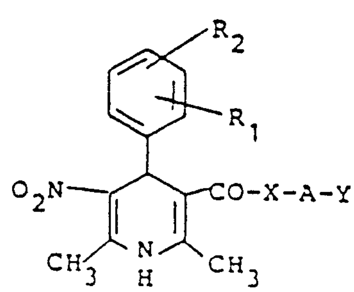

(IX)

in which

$R_1$, $R_2$, X and A have the abovementioned meaning and

Y represents a halogen atom or a reactive group, such as, for example, a mesyl, tosyl or triflate group, are reacted with compounds of the general formula X

$$MeR_3 \quad (X)$$

in which

Me denotes a reactive metal atom, preferably an alkali metal atom, and

$R_3$ represents —S—$COR_6$ or —O—$COR_6$.

5. Process according to Claim 3, characterised in that the reaction is carried out at temperatures between 10 and 150 °C.

6. Medicaments containing at least one compound of the general formula (I) according to Claim 1.

7. Medicaments which influence the circulation and have a positively inotropic action, containing at least one compound of the general formula (I) according to Claim 1.

8. Process for the preparation of medicaments according to Claims 5 and 6, characterised in that compounds of the general formula (I) according to Claim 1 are converted into a suitable form of administration, if appropriate using the usual auxiliaries and excipients.

9. Use of compounds of the general formula (I) according to Claim 1 for the preparation of medicaments for combating circulatory diseases.

**Revendications**

1. Dérivés de 3-nitro-dihydropyridines de formule générale (I)

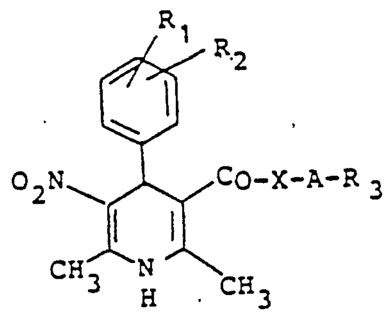

(I)

dans laquelle

$R_1$ représente l'hydrogène,

$R_2$ représente un halogène, un groupe trifluorométhyle, nitro, l'hydrogène, un groupe alkyle en $C_1$ à $C_4$ ou l'un des groupes

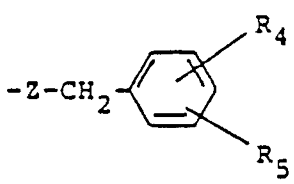

ou

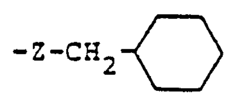

dans lesquels

Z représente l'oxygène ou le soufre et

$R_4$ et $R_5$ sont identiques ou différents et représentent l'hydrogène, un groupe alkyle en $C_1$ à $C_4$, alkoxy en $C_1$ à $C_6$, un halogène, un groupe halogénalkyle en $C_1$ à $C_4$, halogénalkoxy en $C_1$ à $C_4$ ou nitro, ou bien

$R_1$ et $R_2$ forment, conjointement avec deux atomes de carbone du noyau phényle, le noyau

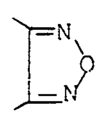

$R_3$ représente un groupe $OCOR_6$, S—$COR_6$, SH, $NH_2$, $COOR_6$, NH—$COR_6$ ou —CO—$NR_7R_8$,

X désigne l'oxygène ou le soufre et

A est un groupe alkylène en $C_2$ à $C_8$,

$R_6$ est l'hydrogène ou un reste aliphatique ayant jusqu'à 6 atomes de carbone ou un reste phényle et

$R_7$ et $R_8$ représentent l'hydrogène ou un reste aliphatique ayant jusqu'à 6 atomes de carbone, ou un reste phényle,

ainsi que leurs sels acceptables du point de vue physiologique.

2. Composés de formule générale (I) suivant la revendication 1, dans laquelle

$R_1$ représente l'hydrogène,

$R_2$ est un halogène, un groupe trifluorométhyle, nitro, un atome d'hydrogène ou l'un des groupes

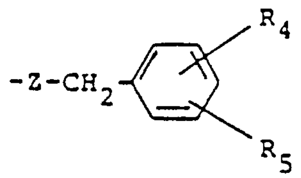 ou 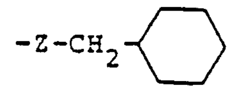

Z, $R_4$ et $R_5$ ayant les définitions déjà indiquées,
$R_3$ est un groupe $OCOR_6$, $S—COR_6$, SH,
X désigne l'oxygène ou le soufre et
A est un groupe alkyle en $C_2$ à $C_8$,
et $R_6$ est un reste alkyle en $C_1$ à $C_3$.

3. Composés de formule générale I suivant la revendication 1, destinés à être utilisés pour combattre des troubles de la circulation.

4. Procédé de production de composés de formule générale (I)

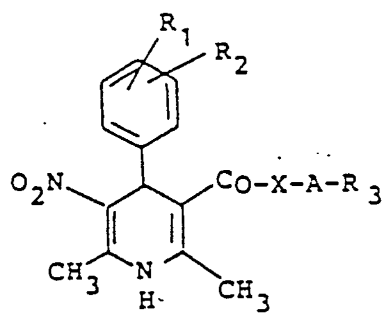 (I)

dans laquelle
$R_1$ représente l'hydrogène,
$R_2$ représente un halogène, un groupe trifluorométhyle, nitro, l'hydrogène, un groupe alkyle en $C_1$ à $C_4$ ou l'un des groupes

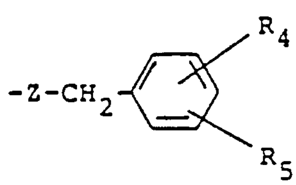 ou 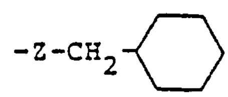

dans lesquels
Z représente l'oxygène ou le soufre et
$R_4$ et $R_5$ sont identiques ou différents et représentent l'hydrogène, un groupe alkyle en $C_1$ à $C_4$, alkoxy en $C_1$ à $C_6$, un halogène, un groupe halogénalkyle en $C_1$ à $C_4$, halogénalkoxy en $C_1$ à $C_4$ ou nitro, ou bien
$R_1$ et $R_2$ forment, conjointement avec deux atomes de carbone du noyau phényle, le noyau

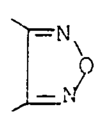

$R_3$ représente un groupe $OCOR_6$, $S—COR_6$, SH, $NH_2$, $COOR_6$, $NH—COR_6$ ou $—CO—NR_7R_8$,
X designe l'oxygène ou le soufre et
A est un groupe alkylène en $C_2$ à $C_8$,
$R_6$ est l'hydrogène ou un reste aliphatique ayant jusqu'à 6 atomes de carbone ou un reste phényle et
$R_7$ et $R_8$ représentent l'hydrogène ou un reste aliphatique ayant jusqu'à 6 atomes de carbone, ou un reste phényle,
ainsi que de leurs sels acceptables du point de vue physiologique, caractérisé en ce que
A) on fait réagir des esters d'acide aminocrotonique de formule générale

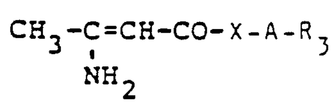 (II)

dans laquelle X, A et $R_3$ ont la définition indiquée ci-dessus, avec des aldéhydes de formule générale (III)

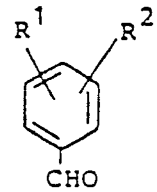

(III)

dans laquelle $R_1$ et $R_2$ ont la définition indiquée ci-dessus, avec la nitroacétone de formule

$$CH_3—CO—CH_2—NO_2$$

ou bien

B) on fait réagir des benzaldéhydes de formule (III) et des esters d'acide acétylacétique de formule générale (IV)

$$CH_3—CO—CH_2—CO—X—A—R_3 \quad (IV)$$

dans laquelle X, A et $R_3$ ont la définition indiquée ci-dessus ou leurs produits de condensation de Knoevenagel (composés ylidéniques) de formule générale (V)

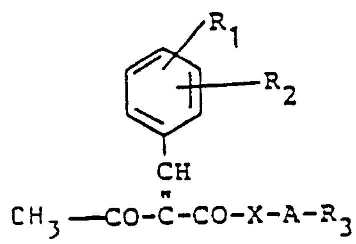

(V)

avec un composé d'addition de la nitro-acétone et de l'ammoniac

$$CH_3—CO—CH_2—NO_2 \cdot NH_3$$

ou bien

C) on fait réagir des esters d'acide aminocrotonique de formule (II) avec des benzylidène-nitro-acétones de formule générale (VI)

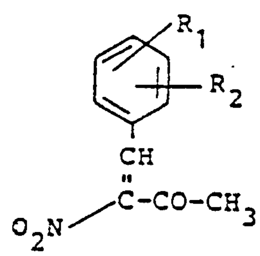

(VI)

ou bien

D) on fait réagir des dérivés d'acides carboxyliques de formule générale

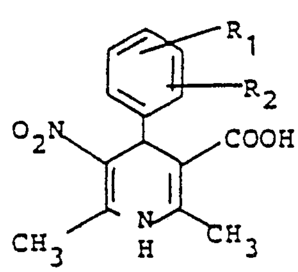

(VII)

par des procédés connus, en passant éventuellement par un dérivé d'acide réactif, avec des composés de formule générale (VIII)

$$H—X—A—R_3 \quad (VIII)$$

dans laquelle X, A et $R_3$ ont la définition indiquée ci-dessus, ou bien

E) on transforme des composés de formule I, dans laquelle $R_1$, $R_2$, X et A ont la définition indiquée ci-dessus et $R_3$ est un groupe —$OCOR_6$, —S—$COR_6$ ou —NH—$COR_6$, par transestérification en composés de formule I dans laquelle $R_3$ représente OH, SH ou —$NH_2$, ou bien

F) on transforme des composés dans lesquels $R_1$, $R_2$, X et A ont la définition indiquée ci-dessus et $R_3$ est un groupe OH, $NH_2$ ou SH, par acylation par des procédés connus en composés dans lesquels $R_3$ est un groupe —O—$COR_6$, —NH—$COR_6$ ou —S—$COR_6$, ou bien

G) pour la préparation de composés de formule générale I dans laquelle $R_1$, $R_2$, X et A ont la définition indiquée ci-dessus et $R_3$ est un groupe —S—$COR_6$ ou —O—$COR_6$,
on fait réagir des composés de formule générale IX

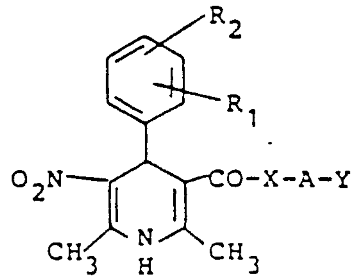

(IX)

dans laquelle

$R_1$, $R_2$, X et A ont la définition indiquée ci-dessus, et

Y représente un atome d'halogène ou un groupe réactif tel que, par exemple, un groupe mésyle, tosyle, triflate,
avec des composés de formule générale X

$$MeR_3 \quad (X)$$

dans laquelle

Me est un atome de métal réactif, de préférence un atome de métal alcalin, et

$R_3$ est un groupe —S—$COR_6$ ou —O—$COR_6$.

5. Procédé suivant la revendication 3, caractérisé en ce que la réaction est conduite à des températures comprises entre 10 et 150 °C.

6. Médicament contenant au moins un composé de formule générale (I) suivant la revendication 1.

7. Médicaments influençant la circulation, à effet inotrope positif, contenant au moins un composé de formule générale (I) suivant la revendication 1.

8. Procédé de préparation de médicaments suivant les revendications 5 et 6, caractérisé en ce qu'on transforme des composés de formule générale (I) suivant la revendication 1 en une forme propre à l'administration, en utilisant éventuellement des adjuvants et des supports classiques.

9. Utilisation de composés de formule générale (I) suivant la revendication 1 pour la préparation de médicaments destinés à combattre des troubles circulatoires.